# EUROPÄISCHE PATENTSCHRIFT

(11) **EP 1 179 339 B1**
(45) Veröffentlichungstag und Bekanntmachung des Hinweises auf die Patenterteilung: **21.03.2007**
(21) Anmeldenummer: 01118508.9
(22) Anmeldetag: 01.08.2001
(51) Int. Cl.: A61Q 5/00, A61Q 5/12, A61Q 19/00, A61Q 19/08, A61K 8/97

(54) **Kosmetische Mittel enthaltend Malvaceae-Samenextrakte**
Cosmetic agent containing extracts of malvaceae seeds
Produit cosmétique contenant des extraits de graines de "malvaceae"

(30) Priorität: 10.08.2000 DE 10039030; 22.12.2000 DE 10064634
(43) Veröffentlichungstag der Anmeldung: 13.02.2002
(73) Patentinhaber: Henkel Kommanditgesellschaft auf Aktien, 40589 Düsseldorf (DE)
(72) Erfinder: Munk, Gabriele, 22763 Hamburg (DE); Poppe, Elisabeth, Dr., 22299 Hamburg (DE); Calas, Marie, 40191 Düsseldorf (DE); Yücel, Sevda, 40591 Düsseldorf (DE); Waldmann-Laue, Marianne, Dr., 40789 Monheim (DE)

(56) Entgegenhaltungen:
- WO-A-00/64278
- WO-A-98/46205
- WO-A-98/53698
- DE-A- 19 738 303
- FR-A- 2 571 256
- DATABASE WPI Derwent Publications Ltd., London, GB; AN 1999-267023 XP002258843 NN: "Preparation of mucous polysaccharides for use in health foods and cosmetics" & JP 11 080203 A (KAGAKU GIJUTSU SHINKO JIGYODAN; KOBE TENNENBUTSU KAGAKU KK)
- KURODA AKIHIRO ET AL: "Skin - lightening sheet packs containing keratinolytic ingredients" CHEMICAL ABSTRACTS + INDEXES, AMERICAN CHEMICAL SOCIETY. COLUMBUS, US, Bd. 133, Nr. 26, 25. Dezember 2000 (2000-12-25), XP002205377 ISSN: 0009-2258
- DUKE J.: "Handbook of Energy Crops (unpublished)" PURDUE UNIVERSITY, [Online] XP002258842 Gefunden im Internet: <URL:http://www.hort.purdue.edu/newcrop/du ke_energy/Hibiscus_sabdariffa.html> [gefunden am 2003-10-22]
- PATENT ABSTRACTS OF JAPAN vol. 008, no. 205 (C-243), 19. September 1984 (1984-09-19) & JP 59 093010 A (ICHIMARU FUARUKOSU KK), 29. Mai 1984 (1984-05-29)
- PATENT ABSTRACTS OF JAPAN vol. 006, no. 186 (C-126), 22. September 1982 (1982-09-22) & JP 57 099517 A (TANPEI SEIYAKU KK), 21. Juni 1982 (1982-06-21)

## Beschreibung

Die Erfindung betrifft kosmetische Mittel, die mindestens einen Malvaceae-Samenextrakt und mindestens ein kationisches Polymer enthalten, zur Behandlung von keratinischen Fasern und Haut.

Kosmetische Mittel zur Pflege und Erhaltung der natürlichen Funktionen von Haut und Haar gewinnen mehr und mehr an Bedeutung. Dazu tragen sowohl die veränderten Verbrauchergewohnheiten und Modetrends als auch die zunehmende Belastung durch Umweltschadstoffe bei. So werden beispielsweise Haut und Haar in ihrer Struktur stärker durch UV-Licht, z. B. durch das intensive Nutzen von Sonnenstudios und die Erhöhung der natürlichen UV-Strahlungsintensität, nachhaltig beeinträchtigt. Diese Beeinträchtigungen zeigen sich auf der Haut wie dem Haar beispielsweise durch einen Verlust der Elastizität.
Weiterhin führt das gesteigerte Hygienebewußtsein der Verbraucher zu einer häufigen intensiven Reinigung von Haut und Haar. Dadurch kann der Schutzfilm aus Talg, welcher kontinuierlich von den zahlreichen Talgdrüsen produziert wird, oder aber die Sebumproduktion der Talgdrüsen selbst stark beeinträchtigt werden. Als Folge kann sich sowohl eine Über- als auch eine Unterproduktion an Sebum einstellen.
Modetrends mit aktuellen Farben für Make-up sowie Haarfärbe- und Haarwellmittel tragen bei beanspruchter Haut und vorbelastetem Haar ein übriges zur Beeinträchtigung des natürlichen Zustandes von Haut und Haar bei. Es ist daher nicht erstaunlich, wenn der Anteil der Verbraucher mit empfindlicher, wenig elastischer, spröder und gereizt reagierender Haut sowie einem in der Kämmbarkeit, dem Glanz, der Elastizität, der Sprödigkeit und der Höchstreißkraft beeinträchtigtem Haar stark zunimmt.
Es hat daher nicht an Versuchen gefehlt, diese Mißstände zu beheben. Dabei wurden u.a. Emulsionen zur Hautpflege bezüglich ihres Reizpotentiales durch die Auswahl geeigneter Emulgatoren weiter optimiert. Zur Reinigung von Haut und Haar werden milde Tenside eingesetzt, um Haut und Haar nicht zusätzlich zu belasten.

Weiterhin finden kosmetische Wirkstoffe zunehmend Verwendung in Mitteln zur Reinigung und Pflege von Oberflächen wie Glas, Porzellan, Leder, Textilien, Fußböden aller Art in Haushalt und Gewerbe, um die Haut des Anwenders derartiger Produkte nicht zusätzlich zu belasten. So sind Handgeschirrspülmitteln mit pflegenden Zusätzen wie Proteinen oder rückfettenden Substanzen im Markt erhältlich.

Es besteht aber weiterhin ein Bedarf an Mitteln, die sich durch eine Verringerung der unerwünschten Beeinträchtigungen von Haut und Haar auszeichnen. Es wurde nunmehr gefunden, daß Malvaceae-Samenextrakte als Wirkstoff in kosmetischen Mitteln, die weiterhin mindestens ein Polymer enthalten, zu überraschend guten Eigenschaften der behandelten Haut und des Haares führen.

Das Dokument WO 98/46205 A1 (Laboratoires Sérobiologiques) beschreibt die Gewinnung von Samenextrakten aus der Malvaceae-Unterart Hibiscus esculentus und ihre Verwendung in kosmetischen Zusammensetzungen. Es offenbart keinen Hinweis auf einen günstigen Effekt bei Kombination mit einem Polymer.

DE 197 38 303 beschreibt die Verwendung einer Wirkstoffkombination bestehend aus einem faserstrukturverbesserndem Wirkstoff und einem Konditionierendem Wirkstoff zum Schutz von Keratinischen Fasern vor Schäden durch Hitze ein wirkung.

Ein erster Gegenstand der Erfindung ist ein kosmetisches Mittel nach Anspruch 1 mit üblichen kosmetischen Rohstoffen, das dadurch gekennzeichnet ist, daß es mindestens einen Samenextrakt von Pflanzen der Gattung Malvaceae und mindestens ein kationisches Polymer enthält, wobei solche Mittel ausgenommen sind, die einen Samenextrakt von Gossypium und ein vernetztes Homopolymer der Acrylsäure enthalten.

Die erfindungsgemäßen Mittel zeigen an vorgeschädigtem Haar eine restrukturierende Wirkung. Weiterhin weist das mit erfindungsgemäßen Mitteln behandelte Haar eine verbesserte mechanische Stabilität, einen verbesserten Griff, mehr Volumen, verbesserten Glanz, verbesserte Kämmbarkeit und verbesserte Elastizität auf. Darüber hinaus wurde eine Schutzwirkung der erfindungsgemäßen Mittel gegenüber einer Haut- oder Haarschädigung durch UV-Strahlen und Ozon gefunden. Die erfindungsgemäßen Mittel können neben der Repair-Wirkung auch präventiven Schutz entfalten.

Ohne an eine Theorie gebunden zu sein, wird vermutet, dass es zu einer Wechselwirkung zwischen den Bestandteilen des Malvaceae-Samenextraktes und dem kationisches Polymer kommt, die sich positiv auf die kosmetischen Eigenschaften der erfindungsgemäßen Mittel auswirkt.

Die im Sinne der Erfindung bevorzugten Pflanzen, die zur Gattung der Malvaceae gehören, sind Baumwolle (Gossypium) mit den Unterarten Gossypium hirsutum, Gossypium barbadense bzw. Gossypium vitifolium, Gossypium herbaceum und Gossypium arboreum, Hibiscus mit den Unterarten Hibiscus esculentus (auch als Okra, Eibisch, Gumbo oder Abelmoschus esculentus bezeichnet), Hibiscus cannabinus (Kenaf), Hibiscus sabdariffa (Rosella), Hibiscus manihot (engl. Sunset Hibiscus) und Hibiscus japonicus sowie Schwarze Malve (Althaea bzw. Alcea rosea, engl. Hollyhock).

Weiterhin geeignet sind die Samen von Sida cordifolia, Sida veronicifolia, Sida ovata, Sida mysorensis, Sida rhombifolia und Abutilon crispum. Zu den besonders bevorzugten Pflanzen zählen Gossypium (Baumwolle) und die Hibiscusunterarten Hibiscus esculentus (Okra), Hibiscus cannabinus (Kenaf) und Hibiscus sabdariffa (Rosella). Ganz besonders bevorzugt sind die Samenextrakte der genannten Baumwollarten mit der INCI-Bezeichnung Gossypium sowie die Samenextrakte von Hibiscus esculentus.

Zur Herstellung der erfindungsgemäß verwendeten Extrakte werden üblicherweise kosmetisch kompatible polare Extraktionsmittel verwendet. Bevorzugt werden Wasser sowie ein- oder mehrwertige C₂₋₄-Alkohole, ausgewählt aus Ethanol, 1-Propanol, 2-Propanol, 1-Butanol, 2-Butanol, Ethylenglykol, 1,2-Propylenglykol, 1,3-Butylenglykol und Glycerin sowie Mischungen hiervon eingesetzt. Besonders bevorzugte Extraktionsmittel sind Wasser, Ethanol, 2-Propanol, 1,2-Propylenglykol, 1,3-Butylenglykol, ganz besonders bevorzugt sind Wasser, Ethanol, 2-Propanol und 1,2-Propylenglykol sowie Mischungen hiervon, z. B. eine Mischung 1,2-Propylenglykol/Wasser im Verhältnis 4:1.

Je nach Wahl der Extraktionsmittel kann es bevorzugt sein, den Malvaceae-Samenextrakt durch Zugabe eines Lösungsvermittlers zu stabilisieren. Als Lösungsvermittler geeignet sind z. B. Ethoxylierungsprodukte von gegebenenfalls gehärteten pflanzlichen und tierischen Ölen. Bevorzugte Lösungsvermittler sind ethoxylierte Mono-, Di- und Triglyceride von C₈₋₂₂-Fettsäuren mit 4 bis 50 Ethylenoxid-Einheiten, z. B. hydriertes ethoxyliertes Castoröl, Olivenölethoxylat, Mandelölethoxylat, Nerzölethoxylat, Polyoxyethylenglycolcapryl-/caprinsäureglyceride, Polyoxyethylenglycerinmonolaurat und Polyoxyethylenglycolkokosfettsäureglyceride. Besonders bevorzugt ist Olivenölethoxylat (INCI-Bezeichnung: PEG-10 Olive Glycerides).

Die Trockenmasse des Extraktes ist abhängig von der Molmasse und der Löslichkeit der dispergierten Inhaltsstoffe und beträgt in der Regel, jeweils bezogen auf das Gewicht des Extraktes, 1 bis 80 Gew.-%. Bevorzugt beträgt die Trockenmasse 15 bis 50 Gew.-% und besonders bevorzugt 20 bis 35 Gew.-%. Bei einem Molekulargewicht der Inhaltsstoffe über 100.000 Dalton kann eine Trockenmasse von 1 bis 20 Gew.-% bevorzugt und eine Trockenmasse von 1 bis 10 Gew.-% besonders bevorzugt sein.
Die erfindungsgemäßen Zusammensetzungen enthalten, bezogen auf ihr Gesamtgewicht, 0,1 - 20 Gew.-% des Malvaceaesamen-Extraktes. Üblicherweise beträgt der Gehalt an Aktivsubstanz mindestens 0,001 Gew.-% und maximal 15 Gew.-%, bezogen auf die Gesamtzusammensetzung. Bevorzugt werden 0,05 - 2 Gew.-% Aktivsubstanz des Malvaceaesamen-Extraktes, besonders bevorzugt 0,1 - 0,5 Gew.-%, jeweils bezogen auf die Gesamtzusammensetzung, eingesetzt.
.Ein besonders bevorzugter Malvaceaesamen-Extrakt stammt aus den Samen von Hibiscus esculentus und ist unter dem Warenzeichen Hibiscin^{®} , z. B. Hibiscin^{®} HP LS 9198 oder Hibiscin^{®} PW LS 9199, von Laboratoires Serobiologiques erhältlich. Ein weiterer besonders bevorzugter Malvaceaesamen-Extrakt stammt aus den Samen von Gossypium und ist z. B. von der Firma Cosmetochem erhältlich.

Die erfindungsgemäß geeigneten Malvaceae-Samenextrakte können nach üblichen Verfahren beispielsweise folgendermaßen hergestellt werden. Die Samenkörner werden geschält und gewünschtenfalls mit konzentrierter Schwefelsäure entfasert. Gegebenenfalls wird die Säure ausgewaschen und der Rückstand getrocknet. Die geschälten, entfaserten Samen werden gemahlen. Anschließend wird das enthaltene Saatöl in mehreren Schritten ausgepreßt. Zur Abtrennung des restlichen Öls wird der Preßkuchen mit einem unpolaren Solvens, z. B. Petrolether, Hexan oder Heptan, nach dem Soxhlet-Verfahren oder einer ähnlichen Prozedur extrahiert. Der Extraktionsrückstand, auch als Schrotmehl bezeichnet, wird getrocknet. Anschließend wird das entölte Schrotmehl mit mindestens einem kosmetisch kompatiblen polaren Lösungsmittel und gegebenenfalls einem Lösungsvermittler nach dem Soxhlet-Verfahren oder einer ähnlichen Prozedur extrahiert. Zur Aufreinigung können gewünschtenfalls mehrere Extraktionsschritte durchgeführt werden. Zuletzt wird ein Teil des Solvens abdestilliert, um den Extrakt auf ein technisch geeignetes Volumen einzuengen.

Die erfindungsgemäß geeigneten Polymere sind ausgewählt aus bestimmten Gruppen von kationischen Polymeren gemäß Anspruch 1, die synthetisch, natürlich oder natürlich mit chemisches oder physikalischen Modifikationen sein können.

Kationische Polymere weisen in der Haupt- und/oder Seitenkette Gruppen auf, die "temporär" oder "permanent" kationisch sein können. Unter "permanent kationisch" werden erfindungsgemäß solche Polymere verstanden, die unabhängig vom pH-Wert des Mittels eine kationische Gruppe aufweisen. Dies sind in der Regel Polymere, deren Monomere eine quartäre Ammoniumgruppe enthalten. Als besonders geeignet haben sich insbesondere solche Polymere erwiesen, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, z. B. die Handelsprodukte Gafquat^{®}.
Als physiologisch verträgliches Gegenionen X⁻ kommen beispielsweise Halogenidionen, Sulfationen, Phosphationen, Methosulfationen sowie organische lonen wie Lactat-, Citrat-, Tartrat- und Acetationen in Betracht. Bevorzugt sind Halogenidionen, insbesondere Chlorid. Ein besonders geeignetes kationisches Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens. Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecylpolyoxypropylenpolyoxyethylenether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/ Dicaprate) und Tridecylpolyoxypropylen-polyoxyethylen-ether im Handel erhältlich.
Weiterhin werden bevorzugt Copolymere eingesetzt, die Methacryloyloxyethyltrimethylammoniumchlorid und als nichtionische Monomereinheiten bevorzugt Acrylamid, Methacrylamid, Acrylsäure-C₁₋₄-alkylester und Methacrylsäure-C₁₋₄-alkylester enthalten. Unter diesen nichtionischen Monomeren ist das Acrylamid besonders bevorzugt. Auch diese Copolymere können, wie für die Homopolymere oben beschrieben, vernetzt sein. Ein erfindungsgemäß bevorzugtes Copolymer ist das vemetzte Acrylamid-Methacryloyloxyethyltrimethylammoniumchlorid-Copolymer. Solche Copolymere, bei denen ionische und nichtionische Monomere in einem Gewichtsverhältnis von etwa 20:80 vorliegen, sind im Handel als ca. 50 %ige nichtwässrige Polymerdispersion unter Namen Salcare^{®} SC 92 erhältlich.
Weitere bevorzugte kationische Polymere sind beispielsweise quaternisierte Cellulosederivate, wie z. B. die Handelsprodukte Celquat^{®} und Polymer JR^{®} , und bevorzugt Celquat^{®} H 100, Celquat^{®} L 200 und Polymer JR^{®} 400, kationische Alkylpolyglycoside gemäß der DE-PS 44 13 686, kationisierter Honig, beispielsweise das Handelsprodukt Honeyquat^{®} 50, kationische Guar-Derivate, wie insbesondere die Produkte Cosmedia^{®} Guar und Jaguar^{®}, Polysiloxane mit quaternären Gruppen, wie z.B. die Handelsprodukte Q2-7224 (Dow Corning), Dow Corning^{®} 929 Emulsion (enthaltend Amodimethicone), SM-2059 (General Electric), SLM-55067 (Wacker) sowie Abil^{®} -Quat 3270 und 3272 (Th. Goldschmidt), polymere Dimethyldiallylammoniumsalze und deren Copolymere mit Estern und Amiden von Acrylsäure und Methacrylsäure, bevorzugt z. B. die Handelsprodukte Merquat^{®} 100 (Poly(dimethyldiallylammoniumchlorid)) und Merquat^{®} 550 (Dimethyldiallylammoniumchlorid-Acrylamid-Copolymer), Copolymere des Vinylpyrrolidons mit quaternierten Derivaten des Dialkylaminoalkylacrylats und -methacrylats, z. B. mit Diethylsulfat quaternierte Vinylpyrrolidon-Dimethylaminoethylmethacrylat-Copolymere. Solche Verbindungen sind als Handelsprodukte Gafquat^{®} 734 und Gafquat^{®} 755 erhältlich, Vinylpyrrolidon-Vinylimidazoliummethochlorid-Copolymere, z. B. die Handelsprodukte Luviquat^{®} FC 370, FC 550, FC 905 und HM 552, quaternierter Polyvinylalkohol, sowie die unter den Bezeichnungen Polyquaternium-2, Polyquaternium-17, Polyquaternium-18 und Polyquaternium-27 bekannten Polymeren mit quartären Stickstoffatomen in der Polymerhauptkette.
Gleichfalls als kationische Polymere eingesetzt werden können die unter den Bezeichnungen Polyquaternium-10 und Polyquaternium-24 bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (ISP), Gafquat^{®} ASCP 1011, Gafquat^{®} HS 110, Luviquat^{®} 8155 und Luviquat^{®} MS 370 erhältlich sind.
Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind Chitosan und dessen Derivate, wie z. B. die Produkte Hydagen^{®} CMF, Hydagen^{®} HCMF, Kytamer^{®} PC und Chitolam^{®} NB/101. Besonders gut geeignete Chitosane weisen einen Deacetylierungsgrad von wenigstens 80 % und ein Molekulargewicht von 5 · 10⁵ bis 5 · 10⁶ g/mol auf. Zur Herstellung erfindungsgemäßer Zubereitungen muß das Chitosan in die Salzform überführt werden. Hierzu geeignete Säuren sind z. B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Die Polymere sind in den erfindungsgemäßen Mitteln bevorzugt in Mengen von 0,01 bis 10 Gew.-%, bezogen auf das gesamte Mittel, enthalten. Mengen von 0,1 bis 5, insbesondere von 0,1 bis 3 Gew.-%, sind besonders bevorzugt.

In einer bevorzugten Ausführungsform enthalten die erfindungsgemäßen Mittel weiterhin mindestens ein Vitamin, Provitamin oder eine Vitaminvorstufe oder deren Derivate. Dabei sind erfindungsgemäß solche Vitamine, Pro-Vitamine und Vitaminvorstufen bevorzugt, die üblicherweise den Gruppen A, B, C, E, F und H zugeordnet werden.

In einer weiteren bevorzugten Ausführungsform enthalten die erfindungsgemäßen Zusammensetzungen mindestens ein Vitamin, Pro-Vitamin, eine Vitaminvorstufe oder deren Derivate. Hierbei sind solche Komponenten bevorzugt, die üblicherweise den Vitamingruppen A, B, C, E, F und H zugeordnet werden.
Zur Gruppe der als Vitamin A-bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäßen Mittel enthalten die Vitamin A-Komponente bevorzugt in Mengen von 0,05 -1 Gew.-%, bezogen auf das gesamte Mittel.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das bevorzugt in Mengen von 0,05 bis 1 Gew.-%, bezogen auf die gesamte Zusammensetzung, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (I) eingesetzt werden.

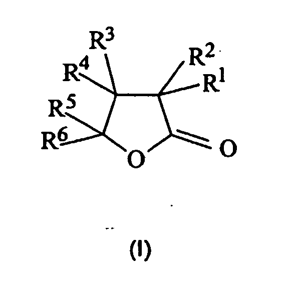

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
Die genannten Verbindungen des Vitamin B₅-Typs sowie die 2-Furanonderivate sind bevorzugt in einer Gesamtmenge von 0,05 bis 10 Gew.-%, besonders bevorzugt 0,1 bis 5 Gew.-%, jeweils bezogen auf die gesamte Zusammensetzung, enthalten.
- Vitamin B₆ (Pyridoxin sowie Pyridoxamin und Pyridoxal).
- Vitamin B₇ (Biotin), auch als Vitamin H oder "Hautvitamin" bezeichnet. Biotin ist bevorzugt in Mengen von 0,0001 bis 1,0 Gew.-%, insbesondere 0,001 bis 0,01 Gew.-%, jeweils bezogen auf die gesamte Zusammensetzung, enthalten.
Vitamin C (Ascorbinsäure) wird bevorzugt in Mengen von 0,1 bis 3 Gew.-%, bezogen auf die gesamte Zusammensetzung, eingesetzt. Die Verwendung der Derivate Ascorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein.
Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.
Unter dem Begriff "Vitamin F" werden üblicherweise essentielle Fettsäuren, insbesondere Linolsäure, Linolensäure und Arachidonsäure, verstanden.
Bevorzugt enthalten die erfindungsgemäßen Mittel Vitamine, Provitamine und Vitaminvorstufen aus den Gruppen A, B, C, E und H. Vitamin A-palmitat (Retinylpalmitat), Panthenol und seine Derivate, Nicotinsäureamid, Ascorbylpalmitat, -acetat, Mg-Ascorbylphosphat, Na-Ascorbylphosphat, Natrium- und Magnesiumascorbat, die Tocopherolester, besonders Tocopherylacetat, und Biotin sind besonders bevorzugt.

In einer weiteren bevorzugten Ausführungsform enthalten die erfindungsgemäßen Mittel oberflächenaktive Substanzen, die je nach Anwendungsgebiet des Mittels als Tenside mit oder als Emulgatoren bezeichnet werden. Als anionische Tenside und Emulgatoren eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside und Emulgatoren sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Acylglutamate mit einem C₈-C₃₀-Acylrest, bevorzugt mit einem C₁₂C₁₈-Acylrest.
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-(O-CH₂-CH₂)ₓ-OSO₃H, in der R eine bevorzugt lineare Alkylgruppe mit 8 bis 30 C-Atomen und x = 0 oder 1 bis 12 ist,
- Gemischte oberflächenaktiver Hydroxysulfonate gemäß DE-A-37 25 030,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Alkyl- und/oder Alkenyletherphosphate,
- sulfatierte Fettsäurealkylenglykolester,
- Monoglyceridsulfate und Monoglyceridethersulfate.
Bevorzugte anionische oberflächenaktiveSubstanzen sind Alkylsulfate, Alkylpolyglykolethersulfate und Ethercarbonsäuren mit C₁₀₋₁₈-Alkylgruppen und bis zu 12 Glykolethergruppen im Molekül und Sulfobernsteinsäuremono- und -dialkylester mit C₈₋₁₈-Alkylgruppen und Sulfobernsteinsäuremonoalkylpolyoxyethylester mit C₈₋₁₆-Alkylgruppen und 1 bis 6 Oxyethylgruppen.

Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, z.B. das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.
Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.
Nichtionische Tenside und Emulgatoren enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- C₁₂-C₃₀-Fettsäuremono- und -diester von Anlagerungsprodukten von 1 bis 30 Mol Ethylenoxid an Polyole mit 3 bis 6 Kohlenstoffatomen, insbesondere an Glycerin,
- Anlagerungsprodukte von 5 bis 60 Mol Ethylenoxid an Rizinusöl und gehärtetes Rizinusöl,
- Polyolfettsäure(partial)ester, wie z.B. Hydagen^{®} HSP (Cognis) oder Sovermol^{®} - Typen (Cognis), insbesondere von gesättigten C₈₋₃₀-Fettsäuren
- alkoxylierte Triglyceride,
- alkoxylierte Fettsäurealkylester,
- Aminoxide,
- Fettsäurealkanolamide, Fettsäure-N-alkylglucamide und Fettamine sowie deren Anlagerungsprodukte von Ethylenoxid und Polyglycerin,
- Sorbitanfettsäureester und Anlagerungsprodukte von Ethylenoxid an Sorbitanfettsäureester wie beispielsweise die Polysorbate,
- Zuckerfettsäureester und Methylglucosid-Fettsäureester sowie deren Anlagerungsprodukte von Ethylenoxid und Polyglycerin,
- Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten.

Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.
Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.
Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.
- Gemische aus Alkyl-(oligo)-glucosiden und Fettalkoholen, z. B. Montanov^{®} 68,
- Sterine, z. B. Ergosterin, Stigmasterin, Sitosterin und Mykosterine,
- Phospholipide, vor allem Glucose-Phospholipide, z. B. Lecithine bzw. Phosphatidylcholine,
- Polyglycerine und Polyglycerinderivate wie beispielsweise Polyglycerinpoly-12-hydroxystearat (Handelsprodukt Dehymuls^{®} PGPH).

Als bevorzugte nichtionische oberflächenaktive Substanzen haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische oberflächenaktive Substanzen Fettsäureester von ethoxyliertem Glycerin enthalten.

In einer bevorzugten Ausführungsform können nichtionische, zwitterionische und/oder amphotere Tenside und Emulgatoren sowie deren Mischungen bevorzugt sein.

Erfindungsgemäß einsetzbar sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationsche Tenside sind die unter den INCI-Bezeichnungen Quaternium-27 und Quaternium-83 bekannten Imidazolium-Verbindungen.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die Produkte Annocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind bevorzugte Beispiele für solche Esterquats.
Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das Stearamidopropyldimethylamin, z.B. das Handelsprodukt Tegoamid^{®} S 18, dar.

Die oberflächenaktiven Substanzen werden in Mengen von 0,1- 45 Gew.%, bevorzugt 0,1 - 30 Gew.% und ganz besonders bevorzugt von 0,5 - 15 Gew.%, bezogen auf das gesamte Mittel, eingesetzt.

Die kationischen Tenside sind in den erfindungsgemäßen Mitteln bevorzugt in Mengen von 0,05 bis 10 Gew.-%, bezogen auf das gesamte Mittel, enthalten. Mengen von 0,1 bis 5 Gew.% sind besonders bevorzugt.

Bevorzugt können die erfindungsgemäßen Zusammensetzungen mindestens einen nichtionischen Emulgator mit einem HLB-Wert von 3 bis 18, gemäß den im Römpp-Lexikon Chemie (Hrg. J. Falbe, M. Regitz), 10. Auflage, Georg Thieme Verlag Stuttgart, New York, (1997), Seite 1764, aufgeführten Definitionen enthalten. Nichtionische O/W-Emulgatoren mit einem HLB-Wert von 10 - 15 sowie nichtionische W/O-Emulgatoren mit einem HLB-Wert von 3 - 6 können erfindungsgemäß besonders bevorzugt sein.

Unter den genannten Emulgatoren-Typen können die Emulgatoren, welche kein Ethylenoxid und/oder Propylenoxid im Molekül enthalten, ganz besonders bevorzugt sein.

In einer speziellen Ausführungsform der Erfindung liegt eine Öl-in-Wasser-Emulsion vor, die frei ist von ionischen sowie von nichtionischen Emulgatoren mit einem HLB-Wert über 6.

Die erfindungsgemäßen Mittel können weiterhin mindestens einen Fettstoff enthalten. Unter Fettstoffen sind Fettsäuren, Fettalkohole, natürliche und synthetische kosmetische Ölkomponenten sowie natürliche und synthetische Wachse zu verstehen, die sowohl in fester Form als auch flüssig in wäßriger Dispersion vorliegen können.
Als Fettsäuren können eingesetzt werden lineare und/oder verzweigte, gesättigte und/oder ungesättigte C₈₋₃₀-Fettsäuren. Bevorzugt sind C₁₀₋₂₂-Fettsäuren. Beispiele sind die lsostearinsäuren und Isopalmitinsäuren wie die unter der Handelsbezeichnung Edenor^{®} vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen. Besonders bevorzugt sind üblicherweise die Fettsäureschnitte, welche aus Cocosöl oder Palmöl erhältlich sind; insbesondere bevorzugt ist der Einsatz von Stearinsäure.
Die Einsatzmenge beträgt dabei 0,1- 15 Gew.%, bezogen auf das gesamte Mittel. In einer bevorzugten Ausführungsform beträgt die Menge 0,5- 10 Gew.%, wobei ganz besonders vorteilhaft Mengen von 1 - 5 Gew.% sind.
Als Fettalkohole können eingesetzt werden gesättigte, ein- oder mehrfach ungesättigte, verzweigte oder unverzweigte Fettalkohole mit 6 - 30, bevorzugt 10 - 22 und ganz besonders bevorzugt 12 - 22 Kohlenstoffatomen. Einsetzbar im Sinne der Erfindung sind z.B. Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll.
Als natürliche oder synthetische Wachse können erfindungsgemäß eingesetzt werden feste Paraffine oder Isoparaffine, Pflanzenwachse wie Candelillawachs, Carnaubawachs, Espartograswachs, Fruchtwachse und Sonnenblumenwachs, Bienenwachse und andere Insektenwachse, Ozokerite, Ceresin, Walrat sowie Microwachse aus Polyethylen oder Polypropylen. Weiterhin geeignet sind die Triglyceride gesättigter und gegebenenfalls hydroxylierter C₁₆₋₃₀-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, weiterhin synthetische Vollester aus Fettsäuren und Glykolen (z. B. Syncrowachs^{®}) oder Polyolen.-mit 2 - 6 C-Atomen, Fettsäuremonoalkanolamide mit einem C₁₂₋₂₂-Acylrest und einem C₂₋₄-Alkanolrest, synthetische Fettsäure-Fettalkoholester, z. B. Stearylstearat oder Cetylpalmitat, Esterwachse aus natürlichen Fettsäuren und synthetischen C₂₀₋₄₀-Fettalkoholen (INCI-Bezeichnung C20-40 Alkyl Stearate) und Vollester aus Fettalkoholen und Di- und Tricarbonsäuren, z. B. Dicetylsuccinat oder Dicetyl-/stearyladipat, sowie Mischungen dieser Substanzen.

Zu den natürlichen und synthetischen kosmetischen Ölkörpern, welche die Wirkung der erfindungsgemäßen Mittel steigern können, sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe wie z. B. 1,3-Di-(2-ethyl-hexyl)-cyclohexan (Cetiol^{®} S) sowie Di-n-alkylether mit insgesamt 12 bis 36, insbesondere 12 bis 24 C-Atomen, wie z. B. Di-n-octylether, Di-n-decylether, Di-n-nonylether, Di-n-undecylether, n-Hexyl-n-octylether und n-Octyl-n-decylether. 1,3-Di-(2-ethylhexyl)-cyclohexan (Cetiol^{®} S) und Di-n-octylether (Cetiol^{®} OE) können bevorzugt sein.
- Esteröle. Unter Esterölen sind zu verstehen die Ester von C₆-₃₀-Fettsäuren mit C₂₋₃₀-Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachidylalkohol, Gadoleylalkohol, Behenylalkohol, Erucylalkohol und Brassidylalkohol sowie technische Mischungen hiervon. Erfindungsgemäß besonders bevorzugt sind Isopropylmyristat, Isononansäure-C₁₆₋₁₈-alkylester, 2-Ethylhexylpalmitat, Stearinsäure-2-ethylhexylester, Cetyloleat, Glycerintricaprylat, Kokosfettalkoholcaprinat/ caprylat, n-Butylstearat, Oleylerucat, Isopropylpalmitat, Oleyloleat, Laurinsäurehexylester, Di-n-butyladipat, Myristylmyristat, Cetearyl Isononanoate und Ölsäuredecylester.
- Dicarbonsäureester wie Di-n-butyladipat, Di-(2-ethylhexyl)-adipat, Di-(2-ethylhexyl) succinat und Di-isotridecylacelaat sowie Diolester wie Ethylenglykoldioleat, Ethylenglykoldi-isotridecanoat, Propylenglykoldi(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Mono,- Di- und Trifettsäureester von gesättigten und/oder ungesättigten linearen und/oder verzweigten Fettsäuren mit Glycerin, wie z. B. Monomuls^{®} 90-O18, Monomuls^{®} 90-L12 oder Cutina^{®} MD.

Die Einsatzmenge beträgt 0,1 - 50 Gew.%, bevorzugt 0,1 - 20 Gew.% und besonders bevorzugt 0,1 - 15 Gew.%, jeweils bezogen auf das gesamte Mittel.
Die Gesamtmenge an Öl- und Fettkomponenten in den erfindungsgemäßen Mitteln beträgt üblicherweise 6 - 45 Gew.-%, bezogen auf das gesamte Mittel. Mengen von 10- 35 Gew.-% sind erfindungsgemäß bevorzugt.
Weiterhin hat sich gezeigt, daß die Wirkung des erfindungsgemäß verwendeten Wirkstoffes gesteigert werden kann, wenn er mit Hydroxycarbonsäureestem kombiniert wird. Bevorzugte Hydroxycarbonsäureester sind Vollester der Glycolsäure, Milchsäure, Äpfelsäure, Weinsäure oder Citronensäure. Weitere grundsätzlich geeignete Hydroxycarbonsäureester sind Ester der β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 - 22 C-Atomen. Dabei sind die Ester von C₁₂-C₁₅-Fettalkoholen besonders bevorzugt. Ester dieses Typs sind z.B. unter dem Warenzeichen Cosmacol^{®} (Eni Chem. Augusta Industriale) erhältlich. Weitere besonders bevorzugte Ölkomponenten sind die Ester von in 2-Position verzweigten C₁₂₋₁₃-Alkanolen mit 2-Ethylhexansäure, z.B. das Handelsprodukt Cosmacol^{®} EOI.
Die Einsatzmenge der Hydroxycarbonsäureester liegt bei 0,1- 15 Gew.%, bevorzugt 0,1 - 10 Gew.% und besonders bevorzugt 0,1 - 5 Gew.%, jeweils bezogen auf das gesamte Mittel.

In einer weiteren Ausführungsform der erfindungsgemäßen Mittel kann die Wirkung durch Verwendung von zusätzlichen Proteinhydrolysaten und deren Derivaten gesteigert werden. Erfindungsgemäß können sowohl pflanzliche als auch tierische Proteinhydrolysate eingesetzt werden. Tierische Proteinhydrolysate sind z.B. Elastin-, Kollagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z.B. DiaMin^{®} (Diamalt), Gluadin^{®} (Cognis), Lexein^{®} (Inolex) und Crotein^{®} (Croda).
Wenngleich der Einsatz der zusätzlichen Proteinhydrolysate als solche bevorzugt ist, können an ihrer Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, z. B. in Form ihrer Fettsäure-Kondensationsprodukte. Entsprechende Handelsprodukte sind z.B. Lamepon^{®} (Cognis), Gluadin^{®} (Cognis), Lexein^{®} (Inolex), Crolastin^{®} oder Crotein^{®} (Croda).
Erfindungsgemäß einsetzbar sind auch kationisierte Proteinhydrolysate, wobei das zugrunde liegende Proteinhydrolysat vom Tier, von der Pflanze, von marinen Lebensformen oder von biotechnologisch gewonnenen Proteinhydrolysaten, stammen kann. Bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen.
Weiterhin können die kationischen Proteinhydrolysate auch noch weiter derivatisiert sein. Als typische Beispiele für erfindungsgemäß verwendete kationische Proteinhydrolysate und - derivate seien einige der unter den INCI - Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte aufgeführt: Cocodimonium Hydroxypropyl Hydrolyzed Collagen, Cocodimonium Hydroxypropyl Hydrolyzed Casein, Steardimonium Hydroxypropyl Hydrolyzed Collagen, Steardimonium Hydroxypropyl Hydrolyzed Hair Keratin, Lauryldimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin. Ganz besonders bevorzugt sind die kationischen Proteinhydrolysate und -derivate auf pflanzlicher Basis.
In den erfindungsgemäß verwendeten Mitteln sind die zusätzlichen Proteinhydrolysate und deren Derivate in Mengen von 0,01 - 10 Gew.-% bezogen auf das gesamte Mittel enthalten. Mengen von 0,1 bis 5 Gew.%, insbesondere 0,1 bis 3 Gew.-%, sind besonders bevorzugt.

Schließlich läßt sich die Wirkung des Wirkstoffes auch durch den kombinierten Einsatz mit weiteren Pflanzenextrakten steigern. Üblicherweise werden diese Extrakte durch Extraktion der gesamten Pflanze hergestellt. Es kann aber in einzelnen Fällen auch bevorzugt sein, die

Extrakte ausschließlich aus Blüten und/oder Blättern der Pflanze oder aber aus den Samen herzustellen.
Erfindungsgemäß sind vor allem die Extrakte aus Grünem Tee, Eichenrinde, Brennessel, Hamamelis, Hopfen, Kamille, Klettenwurzel, Schachtelhalm, Weißdorn, Lindenblüten, Mandel, Kokosnuß, Aloe Vera, Fichtennadel, Roßkastanie, Sandelholz, Wacholder, Mango, Aprikose, Limone, Weizen, Kiwi, Melone, Orange, Grapefruit, Rosmarin, Salbei, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.
Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Kokosnuß, Aloe Vera, Mango, Aprikose, Limone, Weizen, Kiwi, Melone, Orange, Grapefruit, Rosmarin, Salbei, Birke, Wiesenschaumkraut, Quendel, Schafgarbe, Hauhechel, Meristem, Ginseng und Ingwerwurzel. Ganz besonders für die erfindungsgemäße Verwendung geeignet sind die Extrakte aus Grünem Tee, Mandel, Kokosnuß, Aloe Vera, Mango, Aprikose, Limone, Weizen, Kiwi und Melone.
Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.
Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch. Weiterhin kann es bevorzugt sein, in den erfindungsgemäßen Mitteln Mischungen aus mehreren, insbesondere aus zwei, verschiedenen Pflanzenextrakten einzusetzen.

Die erfindungsgemäßen kosmetischen Mittel können neben den weiteren, oben genannten bevorzugten Komponenten prinzipiell alle weiteren, dem Fachmann für solche Mittel bekannten Komponenten enthalten. Weitere Wirk-, Hilfs- und Zusatzstoffe sind beispielsweise Verdickungsmittel wie Gelatine, Johannisbrotkemmehl oder Tone und Schichtsilikate wie z. B. Bentonit, und außerdem Ca-, Mg- oder Zn-Seifen, Strukturanten wie Maleinsäure und Milchsäure, Parfümöle, Dimethylisosorbid, Lösungsmittel und -vermittler wie Ethanol, Isopropanol, Ethylenglykol, Propylenglykol, Glycerin und Diethylenglykol, faserstrukturverbessemde Wirkstoffe, insbesondere Mono-, Di- und Oligosaccharide wie beispielsweise Glucose, Galactose, Fructose, Fruchtzucker, Lactose, Fucose und Rhamnose, quaternierte Amine wie Methyl-1-alkylamidoethyl-2-alkylimidazolinium-methosulfat, Farbstoffe zum Anfärben des Mittels, Antischuppenwirkstoffe wie Piroctone Olamine, Zink Omadine und Climbazol, Lichtschutzmittel, insbesondere Benzophenon-Derivate, Zimtsäure-Derivate und Triazine, Pigmente, Substanzen zur Einstellung des pH-Wertes, z. B. α- und β-Hydroxycarbonsäuren, Wirkstoffe wie Allantoin und Bisabolol, Cholesterin, Komplexbildner wie EDTA, NTA, β-Alanindiessigsäure und Phosphonsäuren, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Ceramide und Pseudoceramide, Trübungsmittel wie Latex, Styrol/PVP- und Styrol/Acrylamid-Copolymere, Perlglanzmittel wie Ethylenglykolmono- und -distearat sowie PEG-3-distearat, Reduktionsmittel wie z. B. Thioglykolsäure und deren Derivate, Thiomilchsäure, Cysteamin, Thioäpfelsäure und α-Mercaptoethansulfonsäure, Treibmittel wie Propan-Butan-Gemische, N₂O, Dimethylether, CO₂ und Luft, Antioxidantien und Pflanzenglycoside.
Bezüglich weiterer fakultativer Komponenten sowie die eingesetzten Mengen dieser Komponenten wird ausdrücklich auf die dem Fachmann bekannten einschlägigen Handbücher, z. B. die Monographie von K. Schrader, Grundlagen und Rezepturen der Kosmetika, 2. Auflage, Hüthig Buch Verlag, Heidelberg, 1989, verwiesen.

Hinsichtlich der Art, gemäß der die erfindungsgemäßen Mittel auf die keratinische Faser, insbesondere das menschliche Haar, sowie auf die Haut aufgebracht wird, bestehen keine prinzipiellen Einschränkungen. Als Konfektionierung dieser Zubereitungen sind beispielsweise Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, grobe, instabile, ein- oder mehrphasige Schüttelmixturen sowie Gele, Sprays, Aerosole und Schaumaerosole geeignet. Diese werden in der Regel auf wäßriger oder wäßrig-alkoholischer Basis formuliert. Als alkoholische Komponente kommen dabei niedere Alkanole sowie Polyole wie Propylenglykol und Glycerin zum Einsatz. Ethanol und Isopropanol sind bevorzugte Alkohole. Wasser und Alkohol können in der wäßrig-alkoholischen Basis in einem Gewichtsverhältnis von 1 : 10 bis 10 : 1 vorliegen. Wasser sowie wäßrig-alkoholische Mischungen, die bis zu 50 Gew.-%, insbesondere bis zu 25 Gew.-%, Alkohol, bezogen auf das Gemisch Alkohol/Wasser, enthalten, können erfindungsgemäß bevorzugte Grundlagen sein. Der pH-Wert dieser Zubereitungen kann prinzipiell bei Werten von 2 - 11 liegen. Er liegt bevorzugt zwischen 2 und 7, wobei Werte von 3 bis 5 besonders bevorzugt sind. Zur Einstellung dieses pH-Wertes kann praktisch jede für kosmetische Zwecke verwendbare Säure oder Base verwendet werden. Üblicherweise werden als Säuren Genußsäuren verwendet, z.B. Essigsäure, Milchsäure, Weinsäure, Zitronensäure, Äpfelsäure, Ascorbinsäure oder Gluconsäure, besonders bevorzugt Zitronensäure oder Milchsäure. Bevorzugte Basen sind Ammoniak, Alkalihydroxide, Monoethanolamin, Triethanolamin sowie N,N,N',N'-Tetrakis-(2-hydroxypropyl)-ethylendiamin.

Auf der Haut und dem Haar verbleibende Zubereitungen haben sich als besonders wirksam erwiesen und können daher bevorzugte Ausführungsformen der erfindungsgemäßen Lehre darstellen. Unter auf der Haut und dem Haar verbleibend werden erfindungsgemäß solche Zubereitungen verstanden, die nicht im Rahmen der Behandlung nach einem Zeitraum von wenigen Sekunden bis zu einer Stunde mit Hilfe von Wasser oder einer wäßrigen Lösung wieder von der Haut ab- oder aus dem Haar ausgespült werden. Vielmehr verbleiben die Zubereitungen bis zur nächsten Wäsche auf der Haut oder dem Haar.

Gemäß einer bevorzugten Ausführungsform für die Anwendung auf dem Haar werden diese Zubereitungen als festigende Mittel oder Styling-Mittel für das Haar wie Haarfestiger, Schaumfestiger, Styling Gels und Fönwellen formuliert. In weiteren bevorzugten Ausführungsformen liegen die erfindungsgemäßen Mittel als Haarspülung, Haarkur oder Haarconditioner vor. Diese können nach Ablauf dieser Einwirkzeit mit Wasser oder einem zumindest überwiegend wasserhaltigen Mittel ausgespült werden; bevorzugt werden sie jedoch auf dem Haar belassen. Dabei kann es bevorzugt sein, die erfindungsgemäße Zubereitung vor der Anwendung eines reinigenden Mittels, eines Wellmittels oder anderen Haarbehandlungsmitteln auf das Haar aufzubringen. .

In einer weiteren bevorzugten Ausführungsform liegen die erfindungsgemäßen Mittel als Hautpflegemittel wie Tagescremes, Nachtcremes oder Gesichtsmasken vor. Bei der Formulierung dieser Mittel, insbesondere bei den O/W-Emulsionen, kann es bevorzugt sein, auf ionische sowie auf nichtionische Emulgatoren mit einem HLB-Wert über 6 zu verzichten.

Gemäß weiteren Ausführungsformen kann es sich bei den erfindungsgemäßen Mitteln aber auch um reinigende Mittel für Haut und Haar wie beispielsweise Shampoos, Gesichtsreiniger, Make-up-Remover, oder um dauerhafte Verformungsmittel wie Dauerwell- und Fixiermittel sowie insbesondere im Rahmen eines Dauerwellverfahrens oder Färbeverfahrens eingesetzte Vorbehandlungsmittel oder Nachspülungen handeln.

In einer besonderen Ausführungsform der erfindungsgemäßen Mittel kann es bevorzugt sein, wenn die Mittel als Mikroemulsion vorliegen. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich im Prinzip um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (üblicherweise als Phaseninversiontemperatur oder "PIT" bezeichnet) Mikroemulsionen aus, die sich bei weiterer Erwärmung in Wasser-in-Öl(W/O)-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Einzelheiten bezüglich dieser sehr stabilen, niedrigviskosen Systeme, für die sich die Bezeichnung "PIT-Emulsionen" allgemein durchgesetzt hat, sind einer Vielzahl von Druckschriften zu entnehmen, für die stellvertretend die Veröffentlichungen in Angew. Chem. 97, 655-669 (1985) und Adv. Colloid Interface Sci 58, 119-149 (1995) genannt werden.

Erfindungsgemäß können solche Mikro- oder "PIT"-Emulsionen bevorzugt sein, die einen mittleren Teilchendurchmesser von etwa 200 nm aufweisen.

In einer weiteren bevorzugten Ausführung wird mindestens ein Malvaceae-Samenextrakt in Mitteln zur oxidativen Aufhellung keratinischer Fasern, z. B. in Blondiermitteln, eingesetzt. In einer weiteren bevorzugten Ausführung wird mindestens ein Malvaceae-Samenextrakt in Mitteln zur dauerhaften Verformung keratinischer Fasern, z. B. in Dauerwellmitteln, eingesetzt.

In einer weiteren bevorzugten Ausführung wird mindestens ein Malvaceae-Samenextrakt in Färbe- oder Tönungsmittel zum Färben keratinischer Fasern eingesetzt. Die Zusammensetzung des Färbe- oder Tönungsmittels unterliegt keinen prinzipiellen Einschränkungen.

Als Färbe- oder Tönungsmittel oder als Farbstoffvorprodukte können
- Oxidationsfarbstoffvorprodukte vom Entwickler- und Kuppler-Typ,
- natürliche und synthetische direktziehende Farbstoffe und
- Vorstufen naturanaloger Farbstoffe, wie Indol- und Indolin-Derivate,
sowie Mischungen von Vertretern einer oder mehrerer dieser Gruppen eingesetzt werden.

Als Oxidationsfarbstoffvorprodukte vom Entwickler-Typ werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Besonders vorteilhafte Entwicklerkomponenten sind p-Phenylendiamin, p-Toluylendiamin, p-Aminophenol, 1-(2'-Hydroxyethyl)-2,5-diaminobenzol, 4-Amino-3-methylphenol. 2-Aminomethyl-4-aminophenol, 2,4,5,6-Tetraaminopyrimidin, 2-Hydroxy-4,5,6-triamino-pyrimidin und 4-Hydroxy-2,5,6-triaminopyrimidin.

Als Oxidationsfarbstoffvorprodukte vom Kuppler-Typ werden in der Regel m-Phenylendiaminderivate, Naphthole, Resorcin und Resorcinderivate, Pyrazolone und m-Aminophenolderivate verwendet. Besonders geeignete Kupplerkomponenten sind 1-Naphthol, 1,5-, 2,7-und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Direktziehende Farbstoffe sind üblicherweise Nitrophenylendiamine, Nitroaminophenole, Azofarbstoffe, Anthrachinone oder Indophenole. Besonders geeignete direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Pikraminsäure, Hydroxyethyl-2-nitro-toluidin, 2-Amino-6-chloro-4-nitrophenol, 4-Ethylamino-3-nitrobenzoesäure und 2-Chloro-6-ethylamino-1-hydroxy-4-nitrobenzol.

In der Natur vorkommende direktziehende Farbstoffe sind beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Als Vorstufen naturanaloger Farbstoffe werden beispielsweise Indole und Indoline sowie deren physiologisch verträgliche Salze verwendet. Bevorzugt werden solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Besonders vorteilhafte Eigenschaften hat vor allem 5,6-Dihydroxyindolin, daneben aber auch N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin sowie N-Methyl-5,6-dihydroxyindol, N-Ethyl-5,6-dihydroxyindol, N-Propyl-5,6-dihydroxyindol und N-Butyl-5,6-dihydroxyindol.

Die Indolin- und Indol-Derivate in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden.

Erfolgt die Ausbildung der eigentlichen Haarfarben im Rahmen eines oxidativen Prozesses, so können übliche Oxidationsmittel, wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Weiterhin kann die Ausbildung der Färbung dadurch unterstützt und gesteigert werden, daß dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

Ein weiterer Gegenstand der vorliegenden Erfindung ist die Verwendung eines kosmetischen Mittels, das mindestens einen Samenextrakt von Pflanzen der Gattung Malvaceae und mindestens ein Polymer enthält, zur Behandlung von keratinischen Fasern, wobei Mittel ausgenommen sind, die einen Samenextrakt von Gossypium und ein vernetztes Homopolymer der Acrylsäure enthalten.
Ein weiterer Gegenstand der vorliegenden Erfindung ist die Verwendung eines kosmetischen Mittels, das mindestens einen Samenextrakt von Pflanzen der Gattung Malvaceae und mindestens ein Polymer enthält, zur Restrukturierung, zum Schutz vor Spliss oder zur mechanischen Stärkung von keratinischen Fasern, wobei Mittel ausgenommen sind, die einen Samenextrakt von Gossypium und ein vernetztes Homopolymer der Acrylsäure enthalten. Ein weiterer Gegenstand der vorliegenden Erfindung ist die Verwendung eines kosmetischen Mittels, das mindestens einen Samenextrakt von Pflanzen der Gattung Malvaceae und mindestens ein Polymer enthält, zur kosmetischen Behandlung menschlicher oder tierischer Haut, wobei Mittel ausgenommen sind, die einen Samenextrakt von Gossypium und ein vernetztes Homopolymer der Acrylsäure enthalten.
Ein weiterer Gegenstand der vorliegenden Erfindung ist die Verwendung eines kosmetischen Mittels, das mindestens einen Samenextrakt von Pflanzen der Gattung Malvaceae und mindestens ein Polymer enthält, zur kosmetischen Behandlung des Erscheinungsbildes der trockenen Haut oder des Erscheinungsbildes der Altershaut, wobei Mittel ausgenommen sind, die einen Samenextrakt von Gossypium und ein vernetztes Homopolymer der Acrylsäure enthalten.

Die nachfolgenden Beispiele sollen den Gegenstand der Erfindung näher erläutern, ohne ihn hierauf zu beschränken.

### Beispiele

### 1. Wirkung der erfindungsgemäßen Haarbehandlungsmittel auf das Haarvolumen

Die Tests zum Nachweis der Wirkung der erfindungsgemäßen Haarbehandlungsmittel auf das Haarvolumen wurden jeweils an mindestens 5 Probanden durchgeführt. Es wurden nur solche Probanden ausgewählt, deren Haar auf beiden Seiten des Kopfes gleich gut kämmbar war. Nachdem das Haar mit Leitungswasser befeuchtet wurde, wurden die Formulierungen aufgetragen. Die Testrezeptur wurde im Halbseitentest gegen eine entsprechende Placeborezeptur, das heißt, eine Rezeptur, in der der Wirkstoff durch eine entsprechende Menge an Wasser ersetzt wurde, geprüft.

Für den Test der Shampooformulierungen wurde das Haar halbseitig, das heißt, jeweils auf einer Kopfseite, mit der erfindungsgemäßen Formulierung bzw. der entsprechenden Placeborezeptur gut gereinigt. Anschließend wurden die Shampoos ausgewaschen und das Haar unter einer Trockenhaube getrocknet.

Für die Tests der Conditioner-Formulierungen sowie von einer Leave-in Kur wurde das Testprocedere folgendermaßen variiert. Zunächst wurde der Kopf ganzseitig mit dem gleichen handelsüblichen Shampoo vorgereinigt. Anschließend erfolgte die Haarbehandlung gemäß der jeweiligen Produktbeschreibung, wobei jedes Produkt halbseitig gegen eine Placeborezeptur getestet wurde.

Die abschließende Bewertung des Haarvolumens und des Griffs wurde von vier haptisch geschulten Fachkräften durchgeführt. Alle Bewertungen wurden unabhängig voneinander abgegeben. Die Halbseitentests ergaben für alle Shampoo-, Conditioning und Styling-Formulierungen eine signifikante Bevorzugung der Mittel, die den Malvaceae-Extrakt enthielten, gegenüber den Mitteln ohne diesen Wirkstoff.

### 2. Wirkung von Hautbehandlungsmittel auf den Feuchtigkeitsgehalt der Haut

Es wurden zwei Cremes in Form von W/O-Emulsionen, die frei waren von Emulgatoren mit einem HLB-Wert über 6, hergestellt. Die Creme I enthielt Hibiscin^{®} HP LS 9198 (ex Laboratoires Serobiologiques), einen Extrakt aus den Samen von Hibiscus esculentus aus der Gattung Malvaceae. Die Creme C enthielt an Stelle des Malvaceae-Samenextraktes das Handelsprodukt V-Protein flüssig (ex Cosmetochem), einen Extrakt aus Erbsen (siehe Tabelle 1).

Die feuchtigkeitsspendende Wirkung der Cremes wurde mittels Corneometrie an 8 Testpersonen in für den Kosmetikfachmann bekannter Weise gemessen. Auf die Haut an der Innenseite der Unterarme wurden jeder Testperson in standardisierter Weise beide Cremes appliziert. 0,5, 1,5, 3, 4,5 und 6 Stunden nach Applikation wurde die relative Hautfeuchtigkeit an den behandelten sowie an unbehandelten Hautpartien mit einem Corneometer gemessen. Die in Tabelle 3 aufgetragenen Corneometer-Werte als Mittelwerte der 8 Testpersonen zeigen, dass die erfindungsgemäße Creme über den gesamten Beobachtungszeitraum eine höhere relative Hautfeuchtigkeit als die Vergleichscreme lieferte.

**Tabelle 1: Zusammensetzung der nicht erfindungsgemäßen Creme I und der Creme C**

| **Bestandteil** | **I [Gew.-%]** | **V [Gew.-%]** |
|---|---|---|
| Distelöl | 3,0 | 3,0 |
| Myritol^{®} PC | 3,5 | 3,5 |
| Lanette^{®} 22 (Behenylalkohol) | 3,0 | 3,0 |
| Glycerinmonostearat | 3,0 | 3,0 |
| Stenol^{®} 16/18 | 2,0 | 2,0 |
| Isopropylstearat | 6,0 | 6,0 |
| Baysilon^{®} M350 | 1,0 | 2,0 |
| Controx^{®} KS | 0,05 | 0,05 |
| 4-Hydroxybenzoesäurepropylester | 0,2 | 0,2 |
| Glycerin | 5,0 | 5,0 |
| 4-Hydroxybenzoesäuremethylester | 0,2 | 0,2 |
| Hibiscin^{®} HP LS 9198 | 3,0 | - |
| V-Protein flüssig COS-1 52122 A | | 9,0 |
| Citronensäure | 0,1 | 0,1 |
| Sepigel^{®} 305 | 2,0 | 2,0 |
| Wasser | ad 100 | ad 100 |

Alle Mengenangaben der Ausgangsprodukte beziehen sich auf die Substanzen in ihrer Handelsform (telle quelle).

### Herstellung der Creme:

Der Malvaceae-Samenextrakt oder das V-Protein flüssig wurden in 60 - 80° C warmem Wasser unter Verwendung eines Homogenisators (Ultraturax) gelöst. Diese Lösung wurde mit der auf 60 - 80° C erwärmten Wasserphase (Wasser, Glycerin, 4-Hydroxybenzoesäuremethylester) unter Rühren vereinigt.

Die Öl- und Fettkomponenten wurden gemischt und auf ca. 80° C erwärmt. Schließlich wurde die Ölphase mit der heißen Wasserphase vereinigt und emulgiert.
Es bildete sich eine stabile Emulsion, nach dem Abkühlen auf 20° C eine glatte Creme von hoher Stabilität, wie die nachfolgend dargestellten Ergebnisse der Lagertests zeigt.

**Tabelle 2: Lagerstabilität der erfindungsgemäßen Creme I**

| **Lagertemperatur Lagerdauer** | **-10°C** | **Raumtemperatur** | **40°C** | **60°C** |
|---|---|---|---|---|
| 4 Wochen | stabil | stabil | stabil | stabil |
| 20 Wochen | nicht stabil | stabil | stabil | stabil |

Die Stabilität der erfindungsgemäßen Emulsion wurde jeweils visuell bestimmt.

**Tabelle 3: Ergebnisse der Corneometer-Messungen (n = 8)**

| **Behandlungszeitraum [h]** | **I [%]** | **C [%]** | **U [%]** |
|---|---|---|---|
| 0,5 | 13,8 | 9,0 | 0,0 |
| 1,5 | 8,8 | 6,0 | 1,1 |
| 3,0 | 8,4 | 6,8 | 0,9 |
| 4,5 | 7,0 | 1,8 | -0,1 |
| 6,0 | 6,4 | 4,5 | 1,9 |

| | | | |
|---|---|---|---|
| I = relative Feuchtigkeit der mit der nicht erfindungsgemäßen Creme I behandelten Haut C = relative Feuchtigkeit der mit der Creme C behandelten Haut U = relative Feuchtigkeit der unbehandelten Haut | | | |

### 3. Rezepturbeispiele

Alle Mengenangaben der Ausgangsprodukte beziehen sich auf die Substanzen in ihrer Handelsform (telle quelle).

### Liste der verwendeten Inhaltsstoffe:

| **Inhaltsstoff** | **Beschreibung** | **INCI-Bezeichnung** |
|---|---|---|
| Ajidew^{®} NL 50 (Ajinomoto) | Pyrrolidoncarbonsäure-Natriumsalz (50 % in Wasser) | Sodium PCA |
| Amisafe^{®} LMA 60 (Ajinomoto) | | Hydroxypropyl Arginine Lauryl/Myristyl Ether HCl |
| Baumwollsamenextrakt (Cosmetochem) | | Propylene Glycol (and) PEG-10 Olive Glycerides (and) Aqua (and) Gossypium |
| Baysilon^{®} M 350 (Bayer) | | Dimethicone |
| Bienenwachs 8100 (Kahl) | | Cera alba |
| Biodocarb^{®} (Milker & Grüning) | 3-lod-2-propinyl-n-butylcarbamat | Iodopropynyl Butylcarbamate |
| Carbopol^{®} 981 (Goodrich) | Polyacrylsäure | Carbomer |
| Celquat^{®} L 200 (DELFT NATIONAL) | quaterniertes Cellulose-Derivat (95 % Aktivsubstanz) | Polyquaternium-4 |
| Cetiol^{®} HE (Cognis) | Polyol-C₈₋₁₆-Fettsäure-Ester | PEG-7 Glyceryl Cocoate |
| CETIOL^{®} J 600 (Cognis) | Flüssiger Wachsester | Oleyl Erucate |
| CETIOL^{®} LC (Cognis) | Capryl-/Caprinsäureester von gesättigten C₁₂₋₁₈-Fettalkoholen | Coco-Caprylate/Caprate |
| CETIOL^{®} OE (Cognis) | Di-n-octyl Ether | Dicaprylyl Ether |
| CETIOL^{®} V (Cognis) | Ölsäuredecylester | Decyl Oleate |
| COMPERLAN^{®} KD (Cognis) | Kokosfettsäurediethanolamid | Cocamide DEA |
| Controx^{®} KS | Tocopherol-Mono-diglyceride-citrat-Gemisch | Tocopherol, Hydrogenated Palm Glycerides Citrate |
| COPHEROL^{®} F 1300 (Cognis) | RRR-(a)-Tocopherol, Aktivsubstanz > 85 % | Tocopherol |
| Cosmedia Guar^{®} C 261 (Cognis) | Guarhydroxypropyltrimethylammoniumchlorid | Guar Hydroxypropyltrimoniumchloride |
| Cremophor^{®} RH40 (BASF) | Rizinusöl-hydriert+40-45-EO | PEG-40 Hydrogenated Castor Oil |
| Crodamol^{®} IPM (Croda) | lsopropylmyristat | Isopropyl Myristate |
| CUTINA^{®} AGS (Cognis) | Ethylenglycoldistearat | Glycol Distearate |
| CUTINA^{®} HR (Cognis) | Gehärtetes Rizinusöl | Hydrogenated Castor Oil |
| DEHYMULS^{®} PGPH (Cognis) | Polyglycerinpoly-12-hydroxystearat | Polyglyceryl-2 Dipolyhydroxystearate |
| DEHYQUART^{®} A (CA) (Cognis) | Cetyltrimethylammonium-chlorid (ca. 25% Aktivsubst.) | Cetrimonium Chloride |
| DEHYQUART^{®} F75 (Cognis) | Fettalkohole-Methyl-triethanolammoniummethylsulfatdialkylester-Gemisch | Distearoylethyl Hydroxyethylmonium Methosulfate (and) Cetearyl Alcohol |
| DEHYQUART^{®} L80 (Cognis) | Propylenglycol-Methyltriethanolammoniummethyl-sulfatdialkylester-Gemisch (ca. 75 % Aktivsubstanz) | Dicocoylethyl Hydroxy-ethylmonium Methosulfate (and) Propylene Glycol |
| DEHYTON^{®} G (Cognis) | N(2-Hydroxyethyl)-N-kokosalkylamidoethyl-carboxymethylglycinat-Natrium-Salz (ca. 30% AS) | Disodium Cocoamphodiacetate |
| DEHYTON^{®} K (Cognis) | Fettsäureamid-Derivat mit Betainstruktur (ca. 30% AS) | Cocamidopropyl Betaine |
| DEHYTON^{®} PK 45 (Cognis) | Fettsäureamid- Derivat mit Betainstruktur (ca. 45% AS) | Cocamidopropyl Betaine |
| Dow Corning^{®} 200 Fluid | Polydimethylsiloxan | Dimethicone |
| EDTA | | Tetrasodium EDTA |
| EMULGADE^{®} PL 68/50 (Cognis) | Gemisch aus Alkylpoly-glycosid und Cetylstearyl-alkohol | Cetearyl Glucoside (and) Cetearyl Alcohol |
| EUMULGIN^{®} B 1 (Cognis) | Polyoxyethylen-12-Cetylstearylalkohol | Ceteareth-12 |
| EUMULGIN^{®} B 2 (Cognis) | Polyoxyethylen-20-Cetylstearylalkohol | Ceteareth-20 |
| EUMULGINO^{®} O5 (Cognis) | Polyoxyethylen-5-Oleylcetylalkohol | Oleth-5 |
| Eumulgin^{®} 010 (Cognis) | Polyoxyethylen-10-Oleylcetylalkohol | Oleth-10 |
| EUPERLAN^{®} PK 1200 (Cognis) | Flüssige Dispersion von perlglanzgebenden pedglanzgebenden Substanzen und Hilfsmittel | Coco Glucoside (and) Glycol Distearate (and) Glycerin |
| EUTANOL^{®} G (Cognis) | 2-Octyldodecanol (Guerbet-Alkohol) | Octyldodecanol |
| Euxyl^{®} K 400 (Schülke & Mayr) | | Methyldibromo Glutaronitrile, Phenoxyethanol - |
| Gafquat^{®} 755N (GAF) | Dimethylaminoethylmethacrylat-Vinylpyrrolidon-Copolymer, mit Diethylsulfat quatemiert (19 % AS/Wasser) | Polyquaternium-11 |
| Gluadin^{®} W 20 (Cognis) | Weizenproteinhydrolysat (20 % AS in Wasser) (20 % AS in Wasser) | Aqua (and) Hydrolyzed Wheat Protein (and) Sodium Benzoate (and) Phenoxy-ethanol (and) Methylparaben (and) Propylparaben) |
| GLUADIN^{®} W 40 (Cognis) | Partialhydrolysat aus Weizen (40 % AS) | Hydrolyzed Wheat Protein |
| GLUADIN^{®} WQ (Cognis) | Kationisiertes Weizen-proteinhydrolysat (ca. 31% AS in Wasser) | Laurdimonium Hydroxy-propyl Hydrolyzed Wheat Protein |
| Hibiscin^{®} HP LS 9198 | Hibiscus esculentus- | Water, Hibiscus esculentus |
| (Laboratoires Sérobiologiques) | Samenextrakt (ca. 5% AS in Wasser) | Seed Extract |
| HYDAGEN^{®} HCMF (Cognis) | Chitosan Pulver | Chitosan |
| Jaguar^{®} HP 105 (Rhodia) | Hydroxypropylgalactomannan | Hydroxypropyl-Guar |
| Keltro^{®} T (Monsanto) | Xanthan-Gum | Xanthan Gum |
| LAMEFORM^{®} TGI (Cognis) | Triglycerindiisostearat | Polyglyceryl-3 Diisostearate |
| LAMEQUAT^{®} L (Cognis) | Kationisiertes Eiweißhydrolysat (ca. 36% AS/Wasser) | Laurdimonium Hydroxypropyl Hydrolyzed Collagen |
| LAMESOFT^{®} PO 65 (Cognis) | Gemisch aus Alkylpolyglycosid und Fettsäuremonoglycerid | Coco Glucoside (and) Glyceryl Oleate |
| LATEKOLL^{®} D (BASF) | Acrylester-Methacrylsäure-Copolymer (25 % AS/Wasser) | Carbomer |
| LIPOCUTIN^{®} (Cognis) | | Aqua (and) Lecithin (and) Cholesterol (and) Decetyl Phosphate |
| Luviskol^{®} K30 (BASF) | Polyvinylpyrrolidon (95 % Aktivsubstanz) | PVP |
| Merquat^{®} 550 (Mobil Oil) | Dimethyldiallylammonium-chlorid-Acrylamid-Copolymer (8 % Aktivsubstanz) | Polyquatemium-7 |
| Myritol^{®} 318 (Cognis) | Capryl-/Caprinsäuretriglycerid. | Caprylic/Capric Triglyceride |
| Myrftol^{®} PC (Cognis) | | Propylene Glycol Dicaprylate/Dicaprate |
| Natrosol^{®} 250 HR (Aqualon) | Hydroxyethylcellulose | Hydroxyethylcellulose |
| NUTRILAN^{®} H (Cognis) | Eiweißpartialhydrolysat (ca. 36% AS/Wasser) | Hydrolyzed Collagen |
| Paraffinöl DAB 9 | | Paraffinum liquidum |
| Phenonip^{®} (NIPA) | | Phenoxyethanol, Methyl-paraben, Ethylparaben, Propylparaben, Butyl-paraben |
| PLANTACARE^{®} 810 UP (Cognis) | C₈₋₁₀-Fettalkoholglycosid (ca. 60% Aktivsubstanz) | Decyl Glucoside |
| PLANTACARE^{®} 818 UP (Cognis) | C₈₋₁₄-Fettalkoholglycosid (ca. 50% Aktivsubstanz) | Coco Glucoside |
| Polymer JR^{®} 400 (Union Carbide) | Quatemisierte Hydroxyethyl-cellulose | Polyquaternium-10 |
| Promois^{®} Milk-CAQ (SEIWA KASEI) | Quatemisiertes Milcheiweiß-Hydrolysat | Cocodimonium Hydroxy-propyl Hydrolyzed Casein |
| Rewoquat^{®} W 575 PG (Witco) | 1-Methyl-2-norpalmalkyl-3-palmfettsäureamidoethylimidazolinium-methosulfat (ca. 75 % Aktivsubstanz in Propylenglykol) | Quaternium-87 (and) Propylene Glycol |
| Salcare^{®} SC96 (Ciba) | Poly(methacryloyloxyethyltrimethylammoniumchlorid), 50%ige nichtwässrige Disp. | Polyquatemium-37, Propylene Glycol, Dicaprylate/ Di-caprate, PPG-1 Trideceth-6 |
| Sepigel^{®} 305 (Seppic) | Poly-2-acrylamido-2-methylpropansulfonat | Polyacrylamide, C13-14 lsoparaffin, Laureth-7 |
| Stenol^{®} 1618 (Cognis) | Cetyl/Stearylalkohol | Cetearyl Alcohol |
| TEXAPON^{®} N 70 (Cognis) | Natriumlaurylethersulfat mit 2 Mol EO (ca. 70% AS) | Sodium Laureth Sulfate |
| TEXAPON^{®} NSO (Cognis) | Natriumolaurylethersulfat (ca. 28 % AS) | Sodium Laureth Sulfate |
| TURPINAL^{®} SL (Cognis) | Acetophosphonsäure (60 % Aktivsubstanz) | Etidronic Acid |
| V-Protein flüssig COS-152/22 A (Cosmetochem) | Erbsenproteinhydrolysat (ca. 2,3 -2,6 % AS) | Water, Propylene Glycol, Hydrolyzed Pea Protein (Pisum Sativum), Methylparaben, Propylparaben, Phenoxyethanol |
| Zincum^{®} N 29 (Bärlocher) | Zinkstearat | Zinc Stearate |

### 1. Shampoo

| **Bestandteil** | **INCI - Bezeichnung** | **Gew.-%** |
|---|---|---|
| TEXAPONG^{®} NSO | Sodium Laureth Sulfate | 40,0 |
| DEHYTON^{®} G | Disodium Cocoampho-diacetate | 6,0 |
| Polymer JR^{®} 400 | Polyquaternium-10 | 0,5 |
| Baumwollsamenextrakt | Propylene Glycol (and) PEG-10 Olive Glycerides (and) Aqua (and) Gossypium | 1,0 |
| Cetiol^{®} HE | PEG-7 Glyceryl Cocoate | 3,0 |
| Citronensäure | Citric Acid | 0,5 |
| Natriumbenzoat | Sodium Benzoate | 0,5 |
| Natriumchlorid | Sodium Chloride | 0,5 |
| Parfüm | | 0,4 |
| Wasser | | ad 100 |

### 2. 2-in-1 Shampoo

| **Bestandteil** | **INCI - Bezeichnung** | **Gew.-%** |
|---|---|---|
| TEXAPON^{®} N 70 | Sodium Laureth Sulfate | 12,0 |
| DEHYTON^{®} PK 45 | Cocamidopropyl Betaine | 2,5 |
| PLANTACARE^{®} 818 UP | Coco Glucoside | 3,0 |
| LAMESOFT^{®} PO 65 | Coco Glucoside (and) Glyceryl Oleate | 3,0 |
| COSMEDIA^{®} GUAR C 261 | Guar Hydroxypropyl Trimonium Chloride | 0,3 |
| EUPERLAN^{®} PK 1200 | Coco-Glucoside (and) Glycol Distearate (and) Glycerin | 5,0 |
| Natriumchlorid | Sodium Chloride | 1,2 |
| Polymer JR^{®} 400 | Polyquaternium-10 | 0,5 |
| Baumwollsamenextrakt | Propylene Glycol (and) PEG-10 Olive Glycerides (and) Aqua (and) Gossypium | 1,0 |
| Euxyl^{®} K 400 | | 1,0 |
| Wasser | | ad 100 |
| | | |
| pH-Wert | | 5,5 |
| Viskosität (mPas), Brookfield RFT, 23°C, Sp.4, 10 Upm | | 6300 |

### 3. Haarspülung

| **Bestandteil** | **INCI-Bezeichnung** | **Gew.-%** |
|---|---|---|
| Eumulgin^{®} B2 | Ceteareth-20 | 0,3 |
| Stenol^{®} 1618 | Cetearyl Alcohol | 3,3 |
| Crodamol^{®} IPM | Isopropyl Myristate | 0,5 |
| Paraffinöl DAB 9 | Paraffinum liquidum | 0,3 |
| Dehyquart^{®} L 80 | Dicocoylethyl Hydroxyethyl-monium Methosulfate (and) Propylene Glycol | 0,4 |
| Cosmedia Guar^{®} C 261 | Guar Hydroxypropyl-trimoniumchloride | 1,5 |
| Promois^{®} Milk-CAQ | Cocodimonium Hydroxypropyl Hydrolyzed Casein | 3,0 |
| Baumwollsamenextrakt | Propylene Glycol (and) PEG-10 Olive Glycerides (and) Aqua (and) Gossypium | 1,0 |
| Citronensäure | Citric Acid | 0,4 |
| Phenonip^{®} | Phenoxyethanol, Methylparaben, Ethylparaben, Propylparaben, Butylparaben | 0,8 |
| Wasser | | ad 100 |

### 4. Haarkur

| **Bestandteil** | **INCI - Bezeichnung** | **Gew.-%** |
|---|---|---|
| Celquat^{®} L 200 | Polyquaternium-4 | 0,6 |
| Luviskol^{®} K30 | PVP | 0,2 |
| D-Panthenol | Panthenol | 0,5 |
| Polymer P1, entsprechend DE 39 29 973 | | 0,6 |
| Dehyquart^{®} A-CA | Cetrimonium Chloride | 1,0 |
| Baumwollsamenextrakt | Propylene Glycol (and) PEG-10 Olive Glycerides (and) Aqua (and) Gossypium | 1,0 |
| Natrosol^{®} 250 HR (Aqualon) | Hydroxyethylcellulose | 1,1 |
| Wasser | | ad 100 |

### 5. Haarkur

| **Bestandteil** | **INCI- Bezeichnung** | **Gew.-%** |
|---|---|---|
| Dehyquart^{®} L80 | Dicocoylethyl Hydroxyethylmonium Methosulfate (and) Propylene Glycol | 2,0 |
| Rewoquat^{®} W 575 PG - | Quatemium-87, Propylene Glycol | 2,0 |
| Paraffinöl DAB 9 | Paraffinum liquidum | 2,0 |
| Stenol 1618 | Cetearyl Alcohol | 6,0 |
| Cosmedia Guar^{®} C 261 | Guar Hydroxypropyl-trimoniumchloride | 0,5 |
| Sepigel^{®} 305 Septgel^{®} 305 | Polyacrylamide, C13-14 Isoparaffin, Laureth-7 | 3,5 |
| Baumwollsamenextrakt | Propylene Glycol (and) PEG-10 Olive Glycerides (and) Aqua (and) Gossypium | 1,0 |
| Ajidew^{®} NL 50 | Sodium PCA | 2,5 |
| Gluadin^{®} W 20 | Aqua (and) Hydrolized Wheat Protein (and) Sodium Benzoate (and) Phenoxyethanol (and) Methylparaben (and) Propylparaben) | 3,0 |
| Citronensäure | Citric Acid | 0,15 |
| Phenonip^{®} | | 0,8 |
| Wasser | | ad 100 |

### 6. Haarkur

| **Bestandteil** | **INCI - Bezeichnung** | **Gew.-%** |
|---|---|---|
| Dehyquart^{®} F75 | Distearoylethyl Hydroxyethylmonium Methosulfate (and) Cetearyl Alcohol | 0,3 |
| Salcare^{®} SC96 | Polyquatemium-37, Propylene Glycol, Dicaprylate/Dicaprate, PPG-1 Trideceth-6 | 5,0 |
| Baumwollsamenextrakt | Propylene Glycol (and) PEG-10 Olive Glycerides (and) Aqua (and) Gossypium | 1,0 |
| Dow Corning^{®} 200 Fluid | Dimethicone | 1,5 |
| Gafquat^{®} 755N | Polyquaternium-11 | 1,5 |
| Biodocarb^{®} | lodopropynyl Butylcarbamate | 0,02 |
| Citronensäure | Citric Acid | 0,15 |
| Parfümöl | | 0,25 |
| Wasser | | ad 100 |

### 7. Haarkur

| **Bestandteil** | **INCI - Bezeichnung** | **Gew.-%** |
|---|---|---|
| Dehyquart^{®} F75 | Distearoylethyl Hydroxyethylmonium Methosulfate (and) Cetearyl Alcohol | 4,0 |
| Stenol^{®} 1618 | Cetearyl Alcohol | 4,0 |
| Paraffinöl DAB 9 | Paraffinum liquidum | 1,5 |
| Dehyquart^{®} A-CA | Cetrimonium Chloride | 4,0 |
| Salcare^{®}SC 96 | Polyquatemium-37, Propylene Glycol, Dicaprylate/ Dicaprate, PPG-1 Trideceth-6 | 1,5 |
| Amisafe-LMA-60^{®} | Hydroxypropyl Arginine Lauryl/Myristyl EtherHCl | 1,0 |
| Gluadin^{®}W 20 | Aqua (and) Hydrolyzed Wheat Protein (and) Sodium Benzoate (and) Phenoxyethanol (and) Methylparaben (and) Propylparaben) | 3,0 |
| Baumwollsamenextrakt (Cosmetochem) | Propylene Glycol (and) PEG-10 Olive Glycerides (and) Aqua (and) Gossypium | 1,0 |
| Zitronensäure | | 0,15 |
| Phenonip^{®} | Phenoxyethanol, Methylparaben, Ethylparaben, Propylparaben, Butylparaben | 0,8 |
| Wasser | | ad 100 |

### 8. Pretreatment-Sprav

| **Bestandteil** | **INCl - Bezeichnung** | **Gew.-%** |
|---|---|---|
| Dehyquart^{®} F75 | Distearoylethyl Hydroxyethylmonium Methosulfate (and) Cetearyl Alcohol | 0,3 |
| Salcare^{®} SC96 | Polyquaternium-37, Propylene Glycol, Dicaprylate/Dicaprate, PPG-1 Trideceth-6 | 0,5 |
| Baumwollsamenextrakt | Propylene Glycol (and) PEG-10 Olive Glycerides (and) Aqua (and) Gossypium | 1,0 |
| Dow Corning^{®} 200 Fluid | Dimethicone | 0,5 |
| Biodocarb^{®} | lodopropynyl Butylcarbamate | 0,02 |
| Parfümöl | | 0,25 |
| Wasser | | ad 100 |

### 9. Schaumfestiger

| **Bestandteil** | **INCI** - **Bezeichnung** | **Gew.-%** |
|---|---|---|
| HYDAGEN^{®} HCMF | Chitosan | 0,4 |
| Glykolsäure (Merck) | Glycolic Acid | 0,2 |
| Baumwollsamenextrakt | Propylene Glycol (and) PEG-10 Olive Glycerides (and) Aqua (and) Gossypium | 1,0 |
| DEHYQUART^{®} A | Cetrimonium Chloride | 1,0 |
| GLUADIN^{®} W 40 | Hydrolyzed Wheat Protein | 2,0 |
| Wasser | | ad 100 |

### 10. Kaltwellfixierung (nicht erfindungsgemäß)

| **Bestandteil** | **INCI - Bezeichnung** | **Gew.-%** |
|---|---|---|
| DEHYTON^{®} K | Cocamidopropyl Betaine | 6,0 |
| NUTRILAN^{®} H | Hydrolyzed Collagen | 5,0 |
| LAMEQUAT^{®} L LAMEQUAT^{®} L | Laurdimonium Hydroxypropyl Hydrolyzed Collagen | 3,0 |
| Wasserstoffperoxid 35%ig | Hydrogen Peroxide | 7,5 |
| Keltrol T (1% Quellung). | Xanthan Gum | 15,0 |
| Baumwollsamenextrakt | Propylene Glycol (and) PEG-10 Olive Glycerides (and) Aqua (and) Gossypium | 1,0 |
| Wasser | | ad 100 |
| | | |
| pH-Wert | | 3,5 |

### 11. Cremehaarfarbe (nicht erfindungsgemäß)

| **Bestandteil** | **INCI - Bezeichnung** | **Gew.-%** |
|---|---|---|
| Stenol^{®} 1618 | Cetearyl Alcohol | 17,0 |
| Baysilon^{®} M 350 | Dimethicone | 0,5 |
| CUTINA^{®} AGS | Glycol Distearate | 1,5 |
| EUMULGIN^{®} B2 | Ceteareth-20 | 3,0 |
| EUMULGIN^{®} B1 | Ceteareth-12 | 3,0 |
| EUMULGIN^{®} 05 | Oleth-5 | 1,0 |
| Eumulgin^{®} 010 | Oleth-10 | 1,0 |
| COMPERLAN^{®} KD | Cocamide DEA | 5,0 |
| DEHYQUART^{®} L 80 | Dicocoylethyl Hydroxyethylmonium Methosulfate (and) Propylene Glycol | 1,5 |
| Propylenglycol | | 5,0 |
| p-Aminophenol | | 0,35 |
| p-Toluylendiamin | | 0,85 |
| 2-Methylresorcin | | 0,14 |
| 6-Methyl-3-aminophenol | | 0,42 |
| Baumwollsamenextrakt | Propylene Glycol (and) PEG-10 Olive Glycerides (and) Aqua (and) Gossypium | 1,0 |
| Natriumsulfit | | 0,6 |
| EDTA | Tetrasodium EDTA | 0,2 |
| Ammoniak, 28% | | 5,0 |
| Wasser | | ad 100 |

### 12. Entwicklerdispersion für Cremehaarfarbe aus Beispiel 11:

| **Bestandteil** | **INCI - Bezeichnung** | **Gew.-%** |
|---|---|---|
| Texapon^{®} NSO | Sodium Laureth Sulfate | 2,1 |
| Wasserstoffperoxid (50%ig) | | 12,0 |
| Turpinal^{®} SL | Etidronic Acid | 1,7 |
| Keltrol T (1% Quellung) | Xanthan Gum | 15,0 |
| Baumwollsamenextrakt | Propylene Glycol (and) PEG-10 Olive Glycerides (and) Aqua (and) Gossypium | 1,0 |
| Gluadin^{®} WQ | Laurdimonium Hydroxypropyl Hydrolyzed Wheat Protein | 0,3 |
| Salcare^{®} SC 96 | Polyquatemium-37, Propylene Glycol, Dicaprylate/ Dicaprate, PPG-1 Trideceth-6 | 1,0 |
| Wasser | | ad 100 |

Die Färbecreme hatte einen pH-Wert von 10,0. Sie bewirkte eine intensive rote Tönung des Haares.

### 13. Cremedauerwelle

### Wellcreme

| **Bestandteil** | **lNCl - Bezeichnung** | **Gew.-%** |
|---|---|---|
| Plantacare^{®} 810 UP | Decyl Glucoside | 5,0 |
| Thioglykolsäure | Thio Glycolic Acid | 8,0 |
| Turpinal^{®} SL | Etidronic Acid | 0,5 |
| Ammoniak (25%ig) | | 7,3 |
| Ammoniumcarbonat | | 3,0 |
| Stenol^{®} 1618 | Cetearyl Alcohol | 5,0 |
| Eutanol^{®} G | Octyldodecanol | 4,0 |
| Baumwollsamenextrakt (Cosmetochem) | Propylene Glycol (and) PEG-10 Olive Glycerides (and) Aqua (and) Gossypium | 1,0 |
| Salcare^{®} SC 96 | Polyquaternium-37, Propylene Glycol, Dicaprylate/ Dicaprate, PPG-1 Trideceth-6 | 3,0 |
| Gluadin^{®} WQ | Laurdimonium Hydroxypropyl Hydrolyzed Wheat Protein | 2,0 |
| Parfümöl | | q.s. |
| Wasser | | ad 100 |

### Fixierlösung

| **Bestandteil** | **INCI - Bezeichnung** | **Gew.-%** |
|---|---|---|
| Plantacare^{®} 810 UP | Decyl Glucoside | 5,0 |
| Cutina^{®} HR | Hydrogenated Castor Oil | 2,0 |
| Kaliumbromat | | 3,5 |
| Nitrilotriessigsäure | | 0,3 |
| Zitronensäure | Citric Acid | 0,2 |
| Baumwollsamenextrakt | Propylene Glycol (and) PEG-10 Olive Glycerides (and) Aqua (and) Gossypium | 1,0 |
| Merquat^{®} 550 | Polyquaternium-7 | 0,5 |
| Hydagen^{®} HCMF | Chitosan | 0,5 |
| Gluadin^{®} WQ | Laurdimonium Hydroxypropyl Hydrolyzed Wheat Protein | 0,5 |
| Parfümöl | | q.s. |
| Wasser | | ad 100 |

### 14. Reichhaltige Nachtpflege (nicht erfindungsgemäß)

| **Bestandteil** | **INCI - Bezeichnung** | **Gew.%** |
|---|---|---|
| EMULGADE^{®} PL 68/50 | Cetearyl Glucoside (and) Cetearyl Alcohol | 3,0 |
| Stenol^{®} 1618 | Cetearyl Alcohol | 4,0 |
| CETIOL^{®} J 600 | Oleyl Erucate | 4,0 |
| CETIOL^{®} V | Decyl Oleate | 4,0 |
| CETIOL^{®} OE | Dicaprylyl Ether | 4,0 |
| MYRITOL^{®} 318 | Caprylic/Capric Triglyceride | 3,5 |
| Baysilon^{®} M 350 | Dimethicone | 0,5 |
| COPHEROL^{®} F 1300 | Tocopherol | 1,0 |
| Glycerin 86 % | Glycerine | 3,0 |
| Keltrol T (1% Quellung) | Xanthan Gum | 15,0 |
| KOH 20 % | | 0,3 |
| LIPOCUTIN^{®} | Aqua (and) Lecithin (and) Cholesterol (and) Decetyl Phosphate | 5,0 |
| Baumwollsamenextrakt | Propylene Glycol (and) PEG-10 Olive Glycerides (and) Aqua (and) Gossypium | 2,0 |
| D-Panthenol USP | | 0,5 |
| Wasser | | ad 100 |

### 15. Reichhaltige W/O Creme (nicht erfindungsgemäß)

| **Bestandteil** | **INCl - Bezeichnung** | **Gew.%** |
|---|---|---|
| DEHYMULS^{®} PGPH | Polyglycerylpoly-12 hydroxystearate | 3,0 |
| LAMEFORM^{®} TGI | Polyglyceryl-3 Diisostearate | 3,0 |
| Bienenwachs 8100 | Cera Alba | 3,0 |
| Baysilon^{®} M 350 | Dimethicone | 0,5 |
| Zincum^{®} N 29 | Zinc Stearate - | 1,0 |
| CETIOL^{®} OE | Dicaprylyl Ether | 3,0 |
| CETIOL^{®} LC | Coco Caprylate/Caprate | 6,0 |
| MYRITOL^{®} 318 | Caprylic/Capric Triglyceride | 8,0 |
| Mandelöl | Almond Oil | 8.0 |
| Baumwollsamenextrakt | Propylene Glycol (and) PEG-10 Olive Glycerides (and) Aqua (and) Gossypium | 1,0 |
| COPHEROC^{®} F 1300 | Tocopherol | 1,0 |
| Glycerin | Glycerine | 5,0 |
| MgSO₄ x 7H₂O | | 1,0 |
| Wasser | | ad 100 |
| Viskosität (mPas) / Brookfield, RVF, 23°C, Spindel TE, 4 Upm, Helipath | | 150000 |

## Patentansprüche

1. Kosmetisches Mittel, enthaltend übliche kosmetische Rohstoffe, **dadurch gekennzeichnet, daß** es mindestens einen Samenextrakt von Pflanzen der Gattung Malvaceae und mindestens ein kationisches Polymer enthält, das ausgewählt ist aus vernetzten und unvernetzten Homo- und Copolymeren mit Monomeren, die eine quartäre Ammoniumgruppe enthalten, Copolymeren mit kationischen und nichtionischen Monomeren, quaternisierten Cellulosederivaten, kationischen Guar-Derivaten, Polysiloxanen mit quaternären Gruppen, polymeren Dimethyldiallylammoniumsalzen und deren Copolymeren mit Estern und Amiden von Acrylsäure und Methacrylsäure, Copolymeren des Vinylpyrrolidons mit quaternierten Derivaten des Dialkylaminoalkylacrylats und -methacrylats, quaterniertem Polyvinylalkohol, Polyquaternium-2. Pofyquaternium-17, Palyquaternium-18, Polyquaternium-27. Polyquaternium-10, Polyquaternium-24 und Chitosan und dessen Derivaten, ausgenommen Mittel, die einen Samenextrakt von Gossypium und ein vernetztes Homopolymer der Acrylsäure enthalten.

2. Kosmetisches Mittel nach Anspruch 1, **dadurch gekennzeichnet, daß** der Samenextrakt von Pflanzen der Gattung Malvaceae stammt, die ausgewählt sind aus Gossypium (Baumwolle), den Hibiscusunterarten Hibiscus esculentus (Okra), Hibiscus cannabinus (Kenaf), Hibiscus sabdariffa (Rosella), Hibiscus manihot und Hibiscus japonicus sowie aus Althaea rosea (Schwarze Malve).

3. Kosmetisches Mittel nach Anspruch 1, **dadurch gekennzeichnet, daß** es mindestens einen Extrakt aus den Samen von Gossypium oder Hibiscus esculentus enthält.

4. Kosmetisches Mittel nach einem der Ansprüche 1 oder 2, **dadurch gekennzeichnet, daß** der Samenextrakt in einer Menge von 0,001 bis 15 Gew.-% Aktivsubstanz, bezogen auf das Gesamtgewicht des Mittels, enthalten ist.

5. Kosmetisches Mittel nach einem der Ansprüche 1 bis 4, **dadurch gekennzeichnet, daß** das Mittel ein Vitamin, ein Provitamin, eine Vitaminvorstufe oder deren Derivate enthält.

6. Kosmetisches Mittel nach einem der Ansprüche 1 bis 5, **dadurch gekennzeichnet, daß** das Mittel mindestens eine oberflächenaktive Substanz enthält.

7. Kosmetisches Mittel nach einem der Ansprüche 1 bis 5, **dadurch gekennzeichnet, daß** das Mittel frei ist von ionischen und nichtionischen oberflächenaktiven Substanzen mit einem HLB-Wert über 6.

8. Verwendung eines Mittels nach einem der Ansprüche 1 bis 7 zur Behandlung von keratinischen Fasern.

9. Verwendung eines Mittels nach einem der Ansprüche 1 bis 7 zur Reparatur, zur Restrukturierung, zum Schutz vor Spliss oder zur mechanischen Stärkung von keratinischen Fasern.

10. Verwendung eines Mittels nach einem der Ansprüche 1 bis 7 zur kosmetischen Behandlung menschlicher oder tierischer Haut.

11. Verwendung eines Mittels nach einem der Ansprüche 1 bis 7 zur kosmetischen Behandlung des Erscheinungsbildes der trockenen Haut oder des Erscheinungsbildes der Altershaut

## Claims

1. Cosmetic agent comprising customary cosmetic raw materials, **characterized in that** it comprises at least one seed extract from plants of the genus Malvaceae and at least one cationic polymer which is chosen from crosslinked and uncrosslinked homopolymers and copolymers with monomers which contain a quaternary ammonium group, copolymers with cationic and nonionic monomers, quaternized cellulose derivatives, cationic guar derivatives, polysiloxanes with quaternary groups, polymeric dimethyldiallylammonium salts and copolymers thereof with esters and amides with acrylic acid and methacrylic acid, copolymers of vinylpyrrolidone with quaternized derivatives of dialkylaminoalkyl acrylate and methacrylate, quaternized polyvinyl alcohol, polyquaternium-2, polyquaternium-17, polyquaternium-18, polyquaternium-27, polyquaternium-10, polyquaternium-24 and chitosan and derivatives thereof, with the exception of agents which comprise a seed extract of Gossypium and a crosslinked homopolymer of acrylic acid.

2. Cosmetic agent according to Claim 1, **characterized in that** the seed extract originates from plants of the genus Malvaceae which are chosen from Gossypium (cotton), the hibiscus subspecies Hibiscus esculentus (okra), Hibiscus cannabinus (kenaf), Hibiscus sabdariffa (rosella), Hibiscus manihot and Hibiscus japonicus, and from Althaea rosea (black mallow).

3. Cosmetic agent according to Claim 1, **characterized in that** it comprises at least one extract from the seeds of Gossypium or Hibiscus esculentus.

4. Cosmetic agent according to one of Claims 1 or 2, **characterized in that** the seed extract is present in an amount of from 0.001 to 15% by weight of active substance, based on the total weight of the agent.

5. Cosmetic agent according to one of Claims 1 to 4, **characterized in that** the agent comprises a vitamin, a provitamin, a vitamin precursor or derivatives thereof.

6. Cosmetic agent according to one of Claims 1 to 5, **characterized in that** the agent comprises at least one surface-active substance.

7. Cosmetic agent according to one of Claims 1 to 5, **characterized in that** the agent is free from ionic and nonionic surface-active substances with an HLB value above 6.

8. Use of an agent according to one of Claims 1 to 7 for the treatment of keratin fibres.

9. Use of an agent according to one of Claims 1 to 7 for the repair, restructuring, protection against splitting and mechanical strengthening of keratin fibres.

10. Use of an agent according to one of Claims 1 to 7 for the cosmetic treatment of human or animal skin.

11. Use of an agent according to one of Claims 1 to 7 for the cosmetic treatment of the appearance of dry skin or the appearance of ageing skin.

## Revendications

1. Agent cosmétique contenant des matières premières cosmétiques usuelles, **caractérisé en ce qu'**il contient au moins un extrait de graines de végétaux du genre Malvaceae et au moins un polymère cationique, qui est choisi parmi les homopolymères et les copolymères réticulés et non réticulés avec des monomères qui contiennent un groupe ammonium quaternaire, les copolymères avec des monomères cationiques et non ioniques, les dérivés de cellulose quaternisés, les dérivés cationiques de la gomme guar, les polysiloxanes avec des groupes quaternaires, les sels polymères du diméthyldiallylammonium et leurs copolymères avec des esters et des amides de l'acide acrylique et méthacrylique, les copolymères de la vinylpyrrolidone avec des dérivés quaternisés de l'acrylate et du méthacrylate de dialkylaminoalkyle, le poly(alcool vinylique) quaternisé, le Polyquaternium-2, le Polyquaternium-17, le Polyquaternium-18, le Polyquaternium-27, le Polyquaternium-10, le Polyquaternium-24 et le chitosane et leurs dérivés, à l'exception des agents qui contiennent un extrait de graines de Gossypium et un homopolymère réticulé de l'acide acrylique.

2. Agent cosmétique selon la revendication 1, **caractérisé en ce que** l'extrait de graines provient de végétaux du genre Malvaceae, qui sont choisis parmi le Gossypium (coton), les sous-types d'hibiscus Hibiscus esculentus (okra), Hibiscus cannabinus (kénaf), Hibiscus sabdariffa (rosella), Hibiscus manihot et Hibiscus japonicus ainsi que l'Althaea rosea (rose trémière).

3. Agent cosmétique selon la revendication 1, **caractérisé en ce qu'**il contient au moins un extrait des graines de Gossypium ou d'Hibiscus esculentus.

4. Agent cosmétique selon l'une quelconque des revendications 1 ou 2, **caractérisé en ce que** l'extrait de graines est contenu en une quantité de 0,001 à 15% en poids de substance active par rapport au poids total de l'agent.

5. Agent cosmétique selon l'une quelconque des revendications 1 à 4, **caractérisé en ce que** l'agent contient une vitamine, une provitamine, un précurseur de vitamine ou leurs dérivés.

6. Agent cosmétique selon l'une quelconque des revendications 1 à 5, **caractérisé en ce que** l'agent contient au moins une substance tensioactive.

7. Agent cosmétique selon l'une quelconque des revendications 1 à 5, **caractérisé en ce que** l'agent est exempt de substances tensioactives ioniques et non ioniques présentant une valeur BHL supérieure à 6.

8. Utilisation d'un agent selon l'une quelconque des revendications 1 à 7 pour le traitement de fibres kératiniques.

9. Utilisation d'un agent selon l'une quelconque des revendications 1 à 7 pour la réparation, la restructuration, la protection contre les fourches ou le renforcement mécanique de fibres kératiniques.

10. Utilisation d'un agent selon l'une quelconque des revendications 1 à 7 pour le traitement cosmétique de la peau humaine ou animale.

11. Utilisation d'un agent selon l'une quelconque des revendications 1 à 7 pour le traitement cosmétique des symptômes de la peau sèche ou des symptômes de la peau vieillissante.
